# EUROPEAN PATENT APPLICATION

(11) **EP 1 985 715 A2**
(43) Date of publication of application: **29.10.2008**
(21) Application number: 08007495.8
(22) Date of filing: 07.07.2005
(51) Int. Cl.: C12Q 1/68

(54) **ESR1 and cervical cancer**

(30) Priority: 16.07.2004 GB 0415988; 16.07.2004 US 588350
(62) Divisional of application: 05774038.3
(71) Applicant: Oncomethylome Sciences, S.A., 4000 Liège (BE)
(72) Inventor: Wisman, Bea, P.O. Box 30001 9700 RB Groningen (NL); Van der Zee, Ate, P.O. Box 30001 9700 RB Groningen (NL)
(74) Representative: Spencer, Matthew Peter

(57) **Abstract**

The present invention provides methods and kits for detecting susceptibility to, or the incidence of, cervical cancer in a sample comprising determining the RNA expression levels of the ESR1 gene, optionally as part of a panel of genes.

SEQUENCE LISTING

<110>  ONCOMETHYLOME SCIENCES S.A.

<120>  ESR1 and cervical cancer

<130>  P72151WO00

<140>  PCT/EP2005/007338
<141>  2005-07-07

<160>  93

<170>  PatentIn version 3.3

<210>  1
<211>  19
<212>  DNA
<213>  Artificial

<220>
<223>  Synthetic Primer

<400>  1
ggcgttcgtt ttgggattg                                                    19


<210>  2
<211>  19
<212>  DNA
<213>  Artificial

<220>
<223>  Synthetic Primer

<400>  2
gccgacacgc gaactctaa                                                    19


<210>  3
<211>  24
<212>  DNA
<213>  Artificial

<220>
<223>  Synthetic Probe


<220>
<221>  misc_feature
<222>  (1)..(1)
<223>  5' end labelled with 6FAM

<220>
<221>  misc_feature
<222>  (24)..(24)
<223>  3' end labelled with TAMRA

<400>  3
cgataaaacc gaacgacccg acga                                              24

<210>  4
<211>  24
<212>  DNA
<213>  Artificial

<220>
<223>  Synthetic Primer

<400>  4
ggatagtcgg atcgagttaa cgtc                                              24


<210>  5
<211>  16
<212>  DNA
<213>  Artificial

<220>
<223>  Synthetic Probe

<400>  5
ccctcccaaa cgccga                                                       16


<210>  6
<211>  28
<212>  DNA
<213>  Artificial

<220>
<223>  Synthetic Primer


<220>
<221>  misc_feature
<222>  (1)..(1)
<223>  5' end labelled with 6FAM

<220>
<221>  misc_feature
<222>  (28)..(28)
<223>  3' end labelled with TAMRA

<400>  6
ttcggtaatt cgtagcggta gggtttgg                                          28


<210>  7
<211>  19
<212>  DNA
<213>  Artificial

<220>
<223>  Synthetic Primer

<400>  7
gaaccaaaac gctccccat                                                    19


<210>  8

<211>  27
<212>  DNA
<213>  Artificial

<220>
<223>  Synthetic Primer

<400>  8
ttatatgtcg gttacgtgcg tttatat                                          27


<210>  9
<211>  22
<212>  DNA
<213>  Artificial

<220>
<223>  Synthetic Probe


<220>
<221>  misc_feature
<222>  (1)..(1)
<223>  5' end labelled with 6FAM

<220>
<221>  misc_feature
<222>  (22)..(22)
<223>  3' end labelled with TAMRA

<400>  9
cccgtcgaaa acccgccgat ta                                               22


<210>  10
<211>  24
<212>  DNA
<213>  Artificial

<220>
<223>  Synthetic Primer

<400>  10
ttattagagg gtggggcgga tcgc                                             24


<210>  11
<211>  21
<212>  DNA
<213>  Artificial

<220>
<223>  Synthetic Primer

<400>  11
gaccccgaac cgcgaccgta a                                                21


<210>  12
<211>  23
<212>  DNA

<213>  Artificial

<220>
<223>  Synthetic Probe


<220>
<221>  misc_feature
<222>  (1)..(1)
<223>  5' end labelled with 6FAM

<220>
<221>  misc_feature
<222>  (23)..(23)
<223>  3' end labelled with TAMRA

<400>  12
agtagtatgg agtcggcggc ggg                                              23


<210>  13
<211>  17
<212>  DNA
<213>  Artificial

<220>
<223>  Synthetic Primer

<400>  13
agttgcgcgg cgatttc                                                     17


<210>  14
<211>  22
<212>  DNA
<213>  Artificial

<220>
<223>  Synthetic Primer

<400>  14
gccccaatac taaatcacga cg                                               22


<210>  15
<211>  23
<212>  DNA
<213>  Artificial

<220>
<223>  Synthetic Probe


<220>
<221>  misc_feature
<222>  (1)..(1)
<223>  5' end labelled with 6FAM

<220>
<221>  misc_feature
<222>  (23)..(23)

<223>  3' end labelled with TAMRA

<400>  15
cggtcgacgt tcggggtgta gcg                                              23


<210>  16
<211>  23
<212>  DNA
<213>  Artificial

<220>
<223>  Synthetic Primer

<400>  16
cgaatatact aaaacaaccc gcg                                              23


<210>  17
<211>  21
<212>  DNA
<213>  Artificial

<220>
<223>  Synthetic Primer

<400>  17
gtatttttc gggagcgagg c                                                 21


<210>  18
<211>  26
<212>  DNA
<213>  Artificial

<220>
<223>  Synthetic Probe


<220>
<221>  misc_feature
<222>  (1)..(1)
<223>  5' end labelled with 6FAM

<220>
<221>  misc_feature
<222>  (26)..(26)
<223>  3' end labelled with TAMRA

<400>  18
aatcctcgcg atacgcaccg tttacg                                           26


<210>  19
<211>  21
<212>  DNA
<213>  Artificial

<220>
<223>  Synthetic Primer

<400>  19
gcgtcggagg ttaaggttgt t                                                21

<210>  20
<211>  22
<212>  DNA
<213>  Artificial

<220>
<223>  Synthetic Primer

<400>  20
ctctccaaaa ttaccgtacg cg                                               22

<210>  21
<211>  19
<212>  DNA
<213>  Artificial

<220>
<223>  Synthetic Probe

<220>
<221>  misc_feature
<222>  (1)..(1)
<223>  5' end labelled with 6FAM

<220>
<221>  misc_feature
<222>  (19)..(19)
<223>  3' end labelled with TAMRA

<400>  21
aactcgctcg cccgccgaa                                                   19

<210>  22
<211>  27
<212>  DNA
<213>  Artificial

<220>
<223>  Synthetic Primer

<400>  22
gggattagaa ttttttatgc gagttgt                                          27

<210>  23
<211>  20
<212>  DNA
<213>  Artificial

<220>
<223>  Synthetic Primer

<400>  23
taccccgacg atacccaaac                                                  20

<210>  24
<211>  22
<212>  DNA
<213>  Artificial

<220>
<223>  Synthetic Probe


<220>
<221>  misc_feature
<222>  (1)..(1)
<223>  5' end labelled with 6FAM

<220>
<221>  misc_feature
<222>  (22)..(22)
<223>  3' end labelled with TAMRA

<400>  24
tgtcgagaac gcgagcgatt cg                                                22


<210>  25
<211>  23
<212>  DNA
<213>  Artificial

<220>
<223>  Synthetic Primer

<400>  25
gttttggaag tatgagggtg acg                                               23


<210>  26
<211>  19
<212>  DNA
<213>  Artificial

<220>
<223>  Synthetic Primer

<400>  26
ttcccgccgc tataaatcg                                                    19


<210>  27
<211>  30
<212>  DNA
<213>  Artificial

<220>
<223>  Synthetic Probe


<220>
<221>  misc_feature
<222>  (1)..(1)

<223>  5' end labelled with 6FAM

<220>
<221>  misc_feature
<222>  (30)..(30)
<223>  3' end labelled with TAMRA

<400>  27
attccgccaa tacacaacaa ccaataaacg                                        30


<210>  28
<211>  30
<212>  DNA
<213>  Artificial

<220>
<223>  Synthetic Primer

<400>  28
cgttatatat cgttcgtagt attcgtgttt                                        30


<210>  29
<211>  18
<212>  DNA
<213>  Artificial

<220>
<223>  Synthetic Primer

<400>  29
ctatcgccgc ctcatcgt                                                     18


<210>  30
<211>  22
<212>  DNA
<213>  Artificial

<220>
<223>  Synthetic Probe


<220>
<221>  misc_feature
<222>  (1)..(1)
<223>  5' end labelled with 6FAM

<220>
<221>  misc_feature
<222>  (22)..(22)
<223>  3' end labelled with TAMRA

<400>  30
cgcgacgtca aacgccacta cg                                                22


<210>  31
<211>  18
<212>  DNA

<213>  Artificial

<220>
<223>  Synthetic Primer

<400>  31
gcgttgaagt cggggttc                                                    18


<210>  32
<211>  24
<212>  DNA
<213>  Artificial

<220>
<223>  Synthetic Primer

<400>  32
cccgtacttc gctaacttta aacg                                             24


<210>  33
<211>  24
<212>  DNA
<213>  Artificial

<220>
<223>  Synthetic Probe


<220>
<221>  misc_feature
<222>  (1)..(1)
<223>  5' end labelled with 6FAM

<220>
<221>  misc_feature
<222>  (24)..(24)
<223>  3' end labelled with TAMRA

<400>  33
acaaacgcga accgaacgaa acca                                             24


<210>  34
<211>  22
<212>  DNA
<213>  Artificial

<220>
<223>  Synthetic Primer

<400>  34
gggcgcgggt ttgggttttt ac                                               22


<210>  35
<211>  22
<212>  DNA
<213>  Artificial

<220>
<223> Synthetic Primer

<400> 35
gaaacaaaaa cccaccgccc cg 22


<210> 36
<211> 25
<212> DNA
<213> Artificial

<220>
<223> Synthetic Probe


<220>
<221> misc_feature
<222> (1)..(1)
<223> 5' end labelled with 6FAM

<220>
<221> misc_feature
<222> (25)..(25)
<223> 3' end labelled with TAMRA

<400> 36
aacgacgccg accccactaa actcc 25


<210> 37
<211> 18
<212> DNA
<213> Artificial

<220>
<223> Synthetic Primer

<400> 37
cgacgcacca acctaccg 18


<210> 38
<211> 25
<212> DNA
<213> Artificial

<220>
<223> Synthetic Primer

<400> 38
gttttgagtt ggttttacgt tcgtt 25


<210> 39
<211> 19
<212> DNA
<213> Artificial

<220>
<223> Synthetic Probe

<220>
<221>  misc_feature
<222>  (1)..(1)
<223>  5' end labelled with 6FAM

<220>
<221>  misc_feature
<222>  (19)..(19)
<223>  3' end labelled with TAMRA

<400>  39
acgccgcgct cacctccct                                                    19


<210>  40
<211>  26
<212>  DNA
<213>  Artificial

<220>
<223>  Synthetic Primer

<400>  40
aattttaggt tagagggtta tcgcgt                                            26


<210>  41
<211>  22
<212>  DNA
<213>  Artificial

<220>
<223>  Synthetic Primer

<400>  41
tccccaaaac gaaactaacg ac                                                22


<210>  42
<211>  19
<212>  DNA
<213>  Artificial

<220>
<223>  Synthetic Probe


<220>
<221>  misc_feature
<222>  (1)..(1)
<223>  5' end labelled with 6FAM

<220>
<221>  misc_feature
<222>  (19)..(19)
<223>  3' end labelled with TAMRA

<400>  42
cgcccacccg acctcgcat                                                    19

<210>  43
<211>  19
<212>  DNA
<213>  Artificial

<220>
<223>  Synthetic Primer

<400>  43
gttaggcggt tagggcgtc                                                    19


<210>  44
<211>  19
<212>  DNA
<213>  Artificial

<220>
<223>  Synthetic Primer

<400>  44
ccgaacgcct ccatcgtat                                                    19


<210>  45
<211>  31
<212>  DNA
<213>  Artificial

<220>
<223>  Synthetic Probe


<220>
<221>  misc_feature
<222>  (1)..(1)
<223>  5' end labelled with 6FAM

<220>
<221>  misc_feature
<222>  (31)..(31)
<223>  3' end labelled with TAMRA

<400>  45
caacatcgtc tacccaacac actctcctac g                                      31


<210>  46
<211>  17
<212>  DNA
<213>  Artificial

<220>
<223>  Synthetic Primer

<400>  46
ggaaaggcgc gtcgagt                                                      17

<210>  47
<211>  18
<212>  DNA
<213>  Artificial

<220>
<223>  Synthetic Primer

<400>  47
tcccctatcc caaacccg                                                    18


<210>  48
<211>  18
<212>  DNA
<213>  Artificial

<220>
<223>  Synthetic Probe


<220>
<221>  misc_feature
<222>  (1)..(1)
<223>  5' end labelled with 6FAM

<220>
<221>  misc_feature
<222>  (18)..(18)
<223>  3' end labelled with TAMRA

<400>  48
cgcgcgtttc ccgaaccg                                                    18


<210>  49
<211>  25
<212>  DNA
<213>  Artificial

<220>
<223>  Synthetic Primer

<400>  49
tggtgatgga ggaggtttag taagt                                            25


<210>  50
<211>  27
<212>  DNA
<213>  Artificial

<220>
<223>  Synthetic Primer

<400>  50
aaccaataaa acctactcct cccttaa                                          27


<210>  51
<211>  30

<212>  DNA
<213>  Artificial

<220>
<223>  Synthetic Probe


<220>
<221>  misc_feature
<222>  (1)..(1)
<223>  5' end labelled with 6FAM

<220>
<221>  misc_feature
<222>  (30)..(30)
<223>  3' end labelled with TAMRA

<400>  51
accaccaccc aacacacaat aacaaacaca                                        30


<210>  52
<211>  25
<212>  DNA
<213>  Artificial

<220>
<223>  Synthetic Primer

<400>  52
tggtgatgga ggaggtttag taagt                                             25


<210>  53
<211>  30
<212>  DNA
<213>  Artificial

<220>
<223>  Synthetic Probe


<220>
<221>  misc_feature
<222>  (1)..(1)
<223>  5' end labelled with 6FAM

<220>
<221>  misc_feature
<222>  (30)..(30)
<223>  3' end labelled with TAMRA

<400>  53
accaccaccc aacacacaat aacaaacaca                                        30


<210>  54
<211>  27
<212>  DNA
<213>  Artificial

<220>
<223>  Sythetic Primer

<400>  54
aaccaataaa acctactcct cccttaa                                          27


<210>  55
<211>  19
<212>  DNA
<213>  Artificial

<220>
<223>  Synthetic Primer

<400>  55
gaaccaaaac gctccccat                                                   19


<210>  56
<211>  22
<212>  DNA
<213>  Artificial

<220>
<223>  Synthetic Probe


<220>
<221>  misc_feature
<222>  (1)..(1)
<223>  5' end labelled with 6FAM

<220>
<221>  misc_feature
<222>  (22)..(22)
<223>  3' end labelled with TAMRA

<400>  56
cccgtcgaaa acccgccgat ta                                               22


<210>  57
<211>  27
<212>  DNA
<213>  Artificial

<220>
<223>  Synthetic Primer

<400>  57
ttatatgtcg gttacgtgcg tttatat                                          27


<210>  58
<211>  17
<212>  DNA
<213>  Artificial


<220>

<223>  Synthetic Primer

<400>  58
ggaaaggcgc gtcgagt                                                    17


<210>  59
<211>  18
<212>  DNA
<213>  Artificial

<220>
<223>  Synthetic Probe


<220>
<221>  misc_feature
<222>  (1)..(1)
<223>  5' end labelled with 6FAM

<220>
<221>  misc_feature
<222>  (18)..(18)
<223>  3' end labelled with TAMRA

<400>  59
cgcgcgtttc ccgaaccg                                                   18


<210>  60
<211>  18
<212>  DNA
<213>  Artificial

<220>
<223>  Synthetic Primer

<400>  60
tcccctatcc caaacccg                                                   18


<210>  61
<211>  23
<212>  DNA
<213>  Artificial

<220>
<223>  Synthetic Primer

<400>  61
gttttggaag tatgagggtg acg                                             23


<210>  62
<211>  30
<212>  DNA
<213>  Artificial

<220>
<223>  Synthetic Probe

<220>
<221> misc_feature
<222> (1)..(1)
<223> 5' end labelled with 6FAM

<220>
<221> misc_feature

<222> (30)..(30)
<223> 3' end labelled with TAMRA

<400> 62
attccgccaa tacacaacaa ccaataaacg 30


<210> 63
<211> 19
<212> DNA
<213> Artificial

<220>
<223> Synthetic Primer

<400> 63
ttcccgccgc tataaatcg 19


<210> 64
<211> 26
<212> DNA
<213> Artificial

<220>
<223> Synthetic Primer

<400> 64
aattttaggt tagagggtta tcgcgt 26


<210> 65

<211> 19
<212> DNA
<213> Artificial

<220>
<223> Synthetic Probe


<220>
<221> misc_feature
<222> (1)..(1)
<223> 5' end labelled with 6FAM

<220>
<221> misc_feature
<222> (19)..(19)
<223> 3' end labelled with TAMRA

<400> 65

cgcccacccg acctcgcat                                                    19


<210>  66
<211>  22
<212>  DNA
<213>  Artificial

<220>
<223>  Synthetic Primer

<400>  66
tccccaaaac gaaactaacg ac                                                 22


<210>  67
<211>  24
<212>  DNA
<213>  Artificial

<220>
<223>  Synthetic Primer

<400>  67
ggatagtcgg atcgagttaa cgtc                                               24


<210>  68
<211>  28
<212>  DNA
<213>  Artificial

<220>
<223>  Synthetic Probe


<220>
<221>  misc_feature
<222>  (1)..(1)
<223>  5' end labelled with 6FAM

<220>
<221>  misc_feature
<222>  (28)..(28)
<223>  3' end labelled with TAMRA

<400>  68
ttcggtaatt cgtagcggta gggtttgg                                           28


<210>  69
<211>  16
<212>  DNA
<213>  Artificial

<220>
<223>  Synthetic Primer

<400>  69
ccctcccaaa cgccga                                                        16

<210>  70
<211>  19
<212>  DNA
<213>  Artificial

<220>
<223>  Synthetic Primer

<400>  70
ggcgttcgtt ttgggattg                                                    19


<210>  71
<211>  24
<212>  DNA
<213>  Artificial

<220>
<223>  Synthetic Probe


<220>
<221>  misc_feature
<222>  (1)..(1)
<223>  5' end labelled with 6FAM

<220>
<221>  misc_feature
<222>  (24)..(24)
<223>  3' end labelled with TAMRA

<400>  71
cgataaaacc gaacgacccg acga                                              24


<210>  72
<211>  19
<212>  DNA
<213>  Artificial

<220>
<223>  Synthetic Primer

<400>  72
gccgacacgc gaactctaa                                                    19


<210>  73
<211>  22
<212>  DNA
<213>  Artificial

<220>
<223>  Synthetic Primer

<400>  73
gggcgcgggt ttgggttttt ac                                                22


<210>  74

<211>  25
<212>  DNA
<213>  Artificial

<220>
<223>  Synthetic Probe


<220>
<221>  misc_feature
<222>  (1)..(1)
<223>  5' end labelled with 6FAM

<220>
<221>  misc_feature
<222>  (25)..(25)
<223>  3' end labelled with TAMRA

<400>  74
aacgacgccg accccactaa actcc                                          25


<210>  75
<211>  22
<212>  DNA
<213>  Artificial

<220>
<223>  Synthetic Primer

<400>  75
gaaacaaaaa cccaccgccc cg                                             22


<210>  76
<211>  19
<212>  DNA
<213>  Artificial

<220>
<223>  Synthetic Primer

<400>  76
gttaggcggt tagggcgtc                                                 19


<210>  77
<211>  31
<212>  DNA
<213>  Artificial

<220>
<223>  Synthetic Probe


<220>
<221>  misc_feature
<222>  (1)..(1)
<223>  5' end labelled with 6FAM

<220>

<221>  misc_feature
<222>  (31)..(31)
<223>  3' end labelled with TAMRA

<400>  77
caacatcgtc tacccaacac actctcctac g                                    31


<210>  78
<211>  19
<212>  DNA
<213>  Artificial

<220>
<223>  Synthetic Primer

<400>  78
ccgaacgcct ccatcgtat                                                  19


<210>  79
<211>  30
<212>  DNA
<213>  Artificial

<220>
<223>  Synthetic Primer

<400>  79
cgttatatat cgttcgtagt attcgtgttt                                      30


<210>  80
<211>  22
<212>  DNA
<213>  Artificial

<220>
<223>  Synthetic Probe


<220>
<221>  misc_feature
<222>  (1)..(1)
<223>  5' end labelled with 6FAM

<220>
<221>  misc_feature
<222>  (22)..(22)
<223>  3' end labelled with TAMRA

<400>  80
cgcgacgtca aacgccacta cg                                              22


<210>  81
<211>  18
<212>  DNA
<213>  Artificial

<220>

<223>  Synthetic Primer

<400>  81
ctatcgccgc ctcatcgt                                                    18


<210>  82
<211>  27
<212>  DNA
<213>  Artificial

<220>
<223>  Synthetic Primer

<400>  82
gggattagaa ttttttatgc gagttgt                                          27


<210>  83
<211>  22
<212>  DNA
<213>  Artificial

<220>
<223>  Synthetic Probe


<220>
<221>  misc_feature
<222>  (1)..(1)
<223>  5' end labelled with 6FAM

<220>
<221>  misc_feature
<222>  (22)..(22)
<223>  3' end labelled with TAMRA

<400>  83
tgtcgagaac gcgagcgatt cg                                               22


<210>  84
<211>  20
<212>  DNA
<213>  Artificial

<220>
<223>  Synthetic Primer

<400>  84
taccccgacg atacccaaac                                                  20


<210>  85
<211>  18
<212>  DNA
<213>  Artificial

<220>
<223>  Synthetic Primer

<400>  85
gcgttgaagt cggggttc                                                    18


<210>  86
<211>  24
<212>  DNA
<213>  Artificial

<220>
<223>  Synthetic Probe


<220>
<221>  misc_feature
<222>  (1)..(1)
<223>  5' end labelled with 6FAM

<220>
<221>  misc_feature
<222>  (24)..(24)
<223>  3' end labelled with TAMRA

<400>  86
acaaacgcga accgaacgaa acca                                             24


<210>  87
<211>  24
<212>  DNA
<213>  Artificial

<220>
<223>  Synthetic Primer

<400>  87
cccgtacttc gctaacttta aacg                                             24


<210>  88
<211>  18
<212>  DNA
<213>  Artificial

<220>
<223>  Synthetic Primer

<400>  88
cgacgcacca acctaccg                                                    18


<210>  89
<211>  19
<212>  DNA
<213>  Artificial

<220>
<223>  Synthetic Probe


<220>

<221>  misc_feature
<222>  (1)..(1)
<223>  5' end labelled with 6FAM

<220>
<221>  misc_feature
<222>  (19)..(19)
<223>  3' end labelled with TAMRA

<400>  89
   acgccgcgct cacctccct                                                    19


<210>  90
<211>  25
<212>  DNA
<213>  Artificial

<220>
<223>  Synthetic Primer

<400>  90
gttttgagtt ggttttacgt tcgtt                                              25


<210>  91
<211>  21
<212>  DNA
<213>  Artificial

<220>
<223>  Synthetic Primer

<400>  91
gcgtcggagg ttaaggttgt t                                                  21


<210>  92
<211>  19
<212>  DNA
<213>  Artificial

<220>
<223>  Synthetic Probe


<220>
<221>  misc_feature
<222>  (1)..(1)
<223>  5' end labelled with 6FAM

<220>
<221>  misc_feature
<222>  (19)..(19)
<223>  3' end labelled with TAMRA

<400>  92
aactcgctcg cccgccgaa                                                     19


<210>  93

<211>  22
<212>  DNA
<213>  Artificial

<220>
<223>  Synthetic Primer

<400>  93
ctctccaaaa ttaccgtacg cg                                                22

## Description

### Field of the invention

The invention relates to methods and kits for use in the detection of cervical cancer which include determination of the methylation status of ESR1.

### Background to the invention

Cervical cancer is a major cause of death in women world wide, which is known to develop from cervical intraepithelial neoplasia (CIN) (1) There is a strong association between certain subtypes (high risk) of the Human Papillomavirus (HPV) and cervical cancer (2). However, it is clear that other factors are also involved in cervical carcinogenesis because the majority of patients infected with HPV will not develop invasive cervical cancer (3).

Cytomorphological examination of cervical smears is the most widely applied screening-method for cervical cancer and its precursors. Disadvantages include the high numbers of false-positive and false-negative cervical smears, leading to an overshoot of diagnostic procedures (4) or a delay in the diagnosis of cervical cancer. False-negative cytology may be found in about 50% of cases when previous negative smears are reviewed from the small proportion of screened women who develop invasive cancer (5,6).

Although it has been suggested that high risk HPV testing may well improve cervical cancer screening (7,8) the specificity for high grade cervical neoplasia of high risk HPV testing is relatively low (9). Therefore new objective diagnostic methods based on molecular changes in cervical cancer are needed.

Silencing of tumour suppressor- or other cancer-associated genes by hypermethylation of CpG islands, located in the promoter and/or 5'-regions of many genes, is a common feature of human cancer (10). CpG island hypermethylation is often associated with a transcriptional block and loss of the relevant protein (10). In addition to the functional implications of gene inactivation in tumour development, these aberrant methylation patterns represent excellent targets for novel diagnostic approaches based on methylation sensitive PCR techniques.

Recently Dong et al. showed that promoter hypermethylation of at least one of the genes p16, DAPK, MGMT, APC, HIC-1, and E-cadherin occurred in 79% of cervical cancer tissues and in none of normal cervical tissues from 24 hysterectomy specimens. Virmani et al. detected aberrant methylation of at least one of the genes p16, RARβ, FHIT, GSTP1, MGMT and hMLH1 in 14 of 19 cervical cancer tissue samples. These experiments were carried out using conventional methylation specific PCR (MSP). In this approach DNA can be amplified using primer pairs designed to distinguish methylated from unmethylated DNA by taking advantage of sequence differences as a result of sodium-bisulfite treatment (11). After sodium-bisulfite treatment unmethylated Cytosine residues are converted to Uracil residues, and methylated Cytosine residues remain unconverted.

An advancement of this technique is called real-time quantitative MSP (QMSP) which permits reliable quantification of methylated DNA. The method is based on the continuous optical monitoring of a fluorogenic PCR. This PCR approach is more sensitive and more specific than conventional PCR and can therefore detect aberrant methylation patterns in human samples with substantial (1:10.000) contamination of normal DNA (12). Moreover, this PCR reaction is amenable to high-throughput techniques allowing the analysis of close to 400 samples in less then 2 hours without requirement for gel-electrophoresis.

Estrogen Receptor Alpha (ESR1) is a gene whose hypermethylation status has been recently shown to be linked to human prostate cancer (Yegnasubramanian et al; Cancer Res. 2004 Mar 15; 64(6): 1975-86). Here rates of hypermethylation were low in prostate cancer tissue and cancer cell lines, but entirely unmethylated in normal tissue. Hypermethylation of ESR1 has also recently been shown to be a useful marker, in combination with other genes, for classifying lung cancers between small cell lung carcinoma (SCLC) and non-small cell lung carcinoma (NSCLC)(Marchevsky et al; J. Mol. Diagn. 2004 Feb; 6(1):28-36). ESR1 mehtylation detection, in combination with other genes, has also been shown to be useful in prognosis of breast cancer (Muller et al; Cancer Res. 2003 Nov 15; 63(22):7641-5) and also, in a panel of 20 genes, for distinguishing different histological stages of eosphageal adenocarcinoma (EAC)(Eads et al; Cancer Res. 2001 Apr 15; 61 (8):3410-8). However, it has not been previously shown or suggested that the hypermethylation status of ESR1 is of value in diagnosing cervical cancer.

### Description of the invention

The present invention provides improved methods to detect cervical cancer in a sample. The analysis herein provided shows for the first time that ESR1 (hyper)methylation is a useful marker for detecting susceptibility to, or the incidence of, cervical cancer in a sample.

Accordingly, in a first aspect of the invention there is provided a method of detecting susceptibility to, or the incidence of, cervical cancer in a sample comprising determining the methylation status of ESR1.

The method is intended to generally relate to an *in vitro* method carried out on an isolated sample.

In a most preferred embodiment, the method comprises determining whether the ESR1 gene is hypermethylated.

Methylation is most commonly associated with promoter regions of genes. Therefore, in most cases methods of detection of methylation will focus on this area of the gene. However, the invention is not limited to only the promoter regions of the relevant genes. If the gene is methylated elsewhere and this methylation is useful in the diagnosis of cervical cancer, it may be included in the methods of the invention, for example by use of appropriate primers and probes.

In the context of the present invention the determination of methylation status will relate to detecting whether specific cytosine residues in the genes under analysis are methylated (to form 5-methylcytosine) or unmethylated. These sites are selected since methylation of these cytosine residues is known to be or predicted to be linked to the incidence of cervical cancer. Thus, in an unaffected individual or one who is less likely to be susceptible to cervical cancer, the relevant residue is unlikely to be methylated or will be methylated at low levels. On the other hand in a susceptible or affected subject the level of methylation at the chosen residues is likely to be significantly increased.

The term "hypermethylated" is well known in the relevant art and refers to aberrant methylation within a gene.

In a most preferred embodiment of the invention, ESR1 forms one of a panel of genes whose methylation status is determined in order to detect susceptibility to, or the incidence of, cervical cancer. There are a number of genes whose methylation status is known to be linked to susceptibility to, or the incidence of, cervical cancer and any of these genes can be potentially included in the method of the invention.

In a preferred embodiment, the method comprises determining the methylation status of any one or more and most preferably all of the panel of genes, preferably determining whether any one of more and most preferably all of the genes are hypermethylated.

In one embodiment of the invention, the panel of genes whose methylation status is determined in order to detect susceptibility to, or the incidence of, cervical cancer also comprises, in addition to ESR1, at least one of the following genes: DAP-kinase, APC, TIMP-3, RAR-beta, CALCA, TSLC1, TIMP-2, DcR1, DcR2, BRCA1, p15, Rassf1A, MLH1, MGMT. Most preferably, the panel will comprise at least DAP-kinase in addition to ESR1.

ESR1 encodes estrogen receptor alpha, a mammary cell receptor. TIMP3 encodes tissue inhibitor of metalloproteinase, and may be involved in angiogenesis. RAR-BETA encodes a tumour suppressor gene which induces apoptosis. CALCA encodes Calcitonin, and is a cell cycle regulator. MLH1 encodes Mut L homolog 1, and is involved in DNA mismatch repair. APC encodes adenomatous polyposis coli; aberrant methylation in the promoter of this gene has been suggested to be a potentially useful biomarker of primary lung cancer (Grote et al; Int. J. Cancer. 2004 Jul 10; 110(5):751-5) .

The TSLC1 gene codes for Tumor Suppressor in Lung cancer 1 (Steenbergen RDM and al. JNCI, 96 (4) : 294-305, 2004). The promoter of TSLC1 has been reported to be hypermethylated in 35% of high-grade CIN lesions and in 58% of squamous cell carcinoma (SCC), as determined in cervical scraping samples. In this study, no hypermethylation was observed in normal cervical biopsies.

TIMP-2 encodes Tissue Inhibitor of Metalloproteinase-2 (Ivanova T and al. Int J Cancer, 108 (6): 882-886, 2004). An MSP assay of TIMP-2 showed in this report aberrant methylation in 47% of invasive cervical carcinoma, while no methylation was observed in the morphologically normal tissue adjacent to the tumors.

DcR1 or DcR2 are TRAIL decoy receptor genes (Shivapurkar N and al., Int J Cancer, 109 (5): 786-792, 2004). Methylation of DcR1 or DcR2 has been shown to be present in 100% of cervical cancer.

The promoter of BRCAL (Breast Cancer 1, Narayan G and al., Molecular Cancer, 2 (24), 2003) has previously been shown to be hypermethylated at a level of 6% in squamous cervical cancers, and in 40% of Adenocarcinomas. In this paper, no methylation was detected in normal tissue.

For the p15 gene (Wong and al., AACR meeting 2003, 44 : #2269), methylation has been observed in 34% of squamous cervical cancer biopsies. Thus, the potential value of these genes for diagnosing cervical cancer is apparent.

p16 is a cell cycle control gene, involved in regulating progression of the cell cycle from G1 to S phase. MGMT (Methyl-Guanine-Methyl-transferase) is an enzyme that functions to repair DNA. GSTP1 encodes a glutathione S-transferase, which are important for detoxifying chemicals that can damage cells. Death-associated Protein (DAP) Kinase plays an important role in apoptotic cell death.

In a previous study (28) the inventors showed that the five tumour suppressor genes p16, DAP-kinase, APC, MGMT and GSTP1 are useful in a method of detecting cervical cancer in a sample because (hyper)methylation of at least one of these genes is seen in 79% of cervical cancer patients and in no control patients.

In one embodiment, the method comprises determining whether any one of (or more than one of) the panel of genes is hypermethylated.

In a particularly preferred aspect of the invention, the method of detecting susceptibility to, or the incidence of, cervical cancer in a sample comprises determining the methylation status of a total of three genes. One of these will be ESR1. The other two genes may be selected from any gene whose methylation status is linked to cervical cancer. For example, the remaining two genes may be selected from the following genes: DAP-kinase, APC, TIMP-3, RAR-beta, CALCA, TSLC1, TIMP-2, DcR1, DcR2, BRCA1, p15, Rassf1A, MLH1, MGMT. Preferably, the remaining two genes whose methylation status is determined (in the sample) will be selected from DAP-Kinase, APC, TIMP-3, CALCA and RAR-beta.

In a further embodiment, the method of detecting susceptibility to, or the incidence of, cervical cancer in a sample comprises determining the methylation status of a total of four genes. One of these genes will be ESR1. The remaining three genes may be selected from any gene whose methylation status is linked to susceptibility to, or the incidence of, cervical cancer. For example, the remaining three genes may be selected from the following genes: DAP-kinase, APC, TIMP-3, RAR-beta, CALCA, TSLC1, TIMP-2, DcR1, DcR2, BRCA1, p15, Rassf1A, MLH1, MGMT. Preferably, the remaining three genes whose methylation status is determined in the sample will be selected from DAP-Kinase, APC, TIMP-3, CALCA and RAR-beta.

In a still further embodiment, the method of detecting susceptibility to, or the incidence of, cervical cancer comprises determining the methylation status of a total of five genes. One of these will be ESR1. The remaining four genes may be selected from any gene whose methylation status is linked to susceptibility to, or the incidence of, cervical cancer. For example, the remaining four genes may be selected from the following genes: DAP-kinase, APC, TIMP-3, RAR-beta, CALCA, TSLC1, TIMP-2, DcR1, DcR2, BRCA1, p15, Rassf1A, MLH1, MGMT. Preferably, the remaining four genes whose methylation status is determined in the sample will be selected from DAP-Kinase, APC, TIMP-3, CALCA and RAR-beta.

In some cases, the genes whose methylation status is linked to susceptibility to, or the incidence of, cervical cancer may not previously have been characterised as such. Candidate genes may be tested using the method of the invention in order to investigate whether their methylation status is linked to, and therefore may improve, the sensitivity of detection of susceptibility to, or the incidence of, cervical cancer. The genes may be tested for whether they are (hyper)methylated in cancer samples. Genes identified in this way may then be added to the panel in order to increase the sensitivity of the detection tests for cervical cancer. A potentially large number of genes may, therefore, be used in the test, to increase sensitivity of the test as long as specificity of the detection method is maintained. These genes may possibly be candidate tumour suppressor genes or other cancer associated genes, where methylation, particularly of CpG islands, may cause a transcriptional block leading to a loss of expression of the functional protein, which in turn may contribute to cervical cancer.

It is preferred that the method of the invention allows at least 80% detection of susceptibility to, or the incidence of, cervical cancer, more preferably at least 85% detection of susceptibility to, or the incidence of, cervical cancer and even more preferably at least 90% detection of susceptibility to, or the incidence of, cervical cancer, most preferably at least 95% detection of susceptibility to, or the incidence of, cervical cancer. An especially preferred method of the invention would utilise a panel of no more than five genes, preferably no more than four genes and even more preferably three genes which, when analysed together, give at least (about) 90% detection of susceptibility to, or the incidence of, cervical cancer.

The method of detecting cervical cancer according to the present invention may be carried out in a multiplex experiment. A multiplex experiment is defined herein as one which allows detection of susceptibility to, or the incidence of, cervical cancer by analysis of the methylation status of a number of genes whose methylation status is linked to susceptibility to, or the incidence of, cervical cancer using a single sample. Multiplexing provides technical advantages because cervical cancer may be accurately diagnosed from a single sample by identifying the methylation status of the whole panel of genes. If many different samples are required for each gene of the panel to be analysed, this may lead to problems of variability between samples, possibly leading to less consistent and accurate detection of cervical cancer. Furthermore, it is preferable for patients if a minimum sample and minimum number of samples are required in order to achieve an accurate diagnosis.

Multiplexing may be performed by using labelled primers, for example by utilising the LUX^{™} fluorogenic primers commercially available from Invitrogen^{™} or as described by Nazarenko et al. NAR 30:e37 (2002) and Nazarenko et al. NAR 30:2089-2095 (2002). This technology is based on labelling and designing at least one of the primers in the primer pair in such a way that it contains a hairpin structure. A fluorescent label is attached to the same primer. Said fluorophore may be "FAM" or "JOE", for example. The hairpin functions as a quencher. The skilled reader would appreciate that alternatives to such probes would work equally well with the invention.

In one embodiment of this aspect of the invention, the methylation status of each of the panel of genes may be detected in a single experiment. This may be the case, for example, if the methylation status of each gene was detected using a probe with a different flurophore molecule attached that fluoresces at a different wavelength. Such fluorophores with different fluorescence emission spectra are well known in the art (also see below). Thus, by way of example, the probes listed in SEQ ID NO's 3, 6, 9, 15, 18, 21, 24, 27, 30, 33, 36, 39, 42 and 45 with the internal reference probe represented by SEQ ID 51, may all have attached a different fluorophore molecule thus permitting the methylation status of a number of genes to be determined in the same single experiment.

Alternatively, the method may employ primers designed to amplify the genes whose methylation status is linked to susceptibility to, or the incidence of, cervical cancer, possibly using PCR, in order to amplify different sized products that may be identified by, for example, separation by size of amplicon using gel electrophoresis. The primers may thus be specific such that they only bind to methylated genes and not to those which are not methylated in the region of interest, which would mean that an amplification product is only formed if the region of interest in the gene of interest is methylated.

It may not, in any case, be necessary to identify exactly which of the aforementioned panel of genes (including ESR1) according to the invention is methylated in order to accurately diagnose susceptibility to, or the incidence of, cervical cancer. Because the panel of genes are selected in order to provide a test where methylation of any one (or a greater number) of the genes indicates the presence of cervical cancer in the sample, so long as one of the genes provides a positive signal for methylation this may be sufficient to detect susceptibility to, or the incidence of, cervical cancer. Methods that rely on an output of positive methylation of at least one of the genes are included within the scope of the invention.

In an alternative embodiment a single sample may be divided into several samples, each of which is used in an individual experiment to detect the methylation status of at least one but not all of the panel of genes whose methylation status is linked to the susceptibility to, or the incidence of, cervical cancer. Therefore, a single sample or portion of a sample may provide the methylation status for each gene which is then used collectively in order to diagnose susceptibility to, or the incidence of, cervical cancer. Each separate experiment may utilise a suitable selection of primers and/or probes required to determine the methylation status of one of the panel of genes.

Advantageously, the method of the invention may decrease the number of false negative results when compared with morphological classification. False negative results are an inherent problem of morphological classification due mainly to the inherent subjectivity of the test. Sampling errors and processing artefacts may also increase the likelihood of false negative results. Furthermore, due to the subjectivity of the test in many cases the significance of the results are not clear cut, and this may lead to a need for regular further testing, including invasive tests.

Furthermore, the invention as described herein may also allow more sensitive detection of susceptibility to, or the incidence of, cervical cancer, requiring less cells in order to achieve an accurate diagnosis. This may have practical benefits for patients where cervical scraping may lead to physical discomfort and repeated testing may be inconvenient.

In one embodiment of the invention determination of the methylation status of the gene(s) is achieved using methylation specific PCR (MSP). MSP is a well known technique for determining the methylation status of a gene sequence. MSP relies upon primer pairs designed to distinguish methylated from unmethylated DNA. The method takes advantage of sequence differences at the primer binding site which are caused by sodium-bisulfite treatment of the sequence (11). Following sodium-bisulfite treatment unmethylated Cytosine residues are converted to Uracil, whereas methylated Cytosine residues (5 methylcytosine) are protected from the action of sodium bisulphite and therefore remain unconverted. Uracil has different base pairing properties to cytosine; uracil corresponds to thymine in its base pairing properties.

The steps of the method of the invention, in one embodiment may be as follows:
a) treat sample with sodium bisulphite,
b) alkaline hydrolysis to convert unmethylated cytosine residues to uracil,
c) amplify sequences of interest using specific primers designed to distinguish between methylated and unmethylated cytosine residues; and
d) detect the products of amplification.

However, the invention is not limited to this series of steps; any method of determining the methylation status of ESR1 and potentially other genes in order to detect susceptibility to, or the incidence of, cervical cancer in a sample is within the scope of the invention.

The suitable design of the primers depends upon the sequence of ESR1 and potentially other genes which form the subject of the analysis and the location of the methylation sites within these genes. The design of suitable primer sequences is well within the capability of one of skill in the art. In a preferred embodiment, the methylation status of ESR1 is determined using primers comprising, consisting essentially of or consisting of the sequence of SEQ ID NO:1 (forward primer)and SEQ ID NO:2 (reverse primer). If, for example, the method also comprises determining the methylation status of DAP-kinase, then primers comprising, consisting essentially of or consisting of the sequence of SEQ ID NO: 4 (forward primer) and SEQ ID NO: 5 (reverse primer) may be utilised.

The primers described herein are methylation specific primers in that they will direct amplification of a sequence where the appropriate cytosine residues are methylated and therefore protected from bisulphite treatment. Thus, the presence of an amplification product indicates the presence of methylation at the cytosine residue and therefore a susceptibility to, or incidence of, cervical cancer.

In a preferred embodiment, the method of the invention is a method of diagnosing susceptibility to, or the incidence of, cervical cancer in a sample wherein the methylation status of the gene(s) is determined using quantitative methylation specific PCR (QMSP). The method in this embodiment, therefore, relies upon use of QMSP to achieve real time quantification of methylation levels of certain gene sequences. QMSP is a technique well characterised in the art for real time detection of the methylation status of genes. The method is an extension of the MSP method and, like MSP, relies upon the fact that following sodium-bisulfite treatment unmethylated Cytosine residues are converted to Uracil residues, and methylated Cytosine residues remain unconverted. By designing suitable primers that take advantage of the sequence differences, methylated genes may be distinguished from those that are not methylated.

QMSP is based on the continuous optical monitoring of a fluorogenic PCR. Therefore, in addition to the ("MSP") primers a fluorescent probe is required. In a preferred embodiment of the invention a Taqman^{®} (Applied Biosystems) probe is used. Such probes are widely commercially available, and the Taqman^{®} system (Applied Biosystems) is well known in the art. Taqman^{®} probes anneal between the upstream and downstream primer in a PCR reaction. They contain a 5'-fluorophore and a 3'-quencher. During amplification, the 5'-3' exonuclease activity of the Taq polymerase cleaves the fluorophore off the probe. Since the fluorophore is no longer in close proximity to the quencher, the fluorophore will be allowed to fluoresce. The resulting fluorescence may be measured, and is in direct proportion to the amount of target sequence that is being amplified.

However, the skilled reader will appreciate that alternatives to such probes would work equally with the invention. For example, in a separate embodiment of the invention, molecular beacon technology may be employed. In this system the beacons are hairpin-shaped (stem-loop) probes with an internally quenched fluorophore whose fluorescence is restored when bound to its target. The loop portion acts as the probe while the stem is formed by complimentary "arm" sequences at the ends of the beacon. A fluorophore and quenching moiety are attached at opposite ends, the stem keeping each of the moieties in close proximity, causing the fluorophore to be quenched by energy transfer. When the beacon detects and binds to its target, it undergoes a conformational change forcing the stem apart, thus separating the fluorophore and quencher. This causes the energy transfer to be disrupted to restore fluorescence.

A further real-time fluorescence based system which may be incorporated in the methods of the invention is Zeneca's Scorpion system, see Detection of PCR products using self-probing amplicons and fluorescence by Whitcombe et al. Nature Biotechnology 17, 804 - 807 (01 Aug 1999). This reference is incorporated into the application in its entirety. The method is based on a primer with a tail attached to its 5' end by a linker that prevents copying of the 5' extension. The probe element is designed so that it hybridizes to its target only when the target site has been incorporated into the same molecule by extension of the tailed primer. This method produces a rapid and reliable signal, because probe-target binding is kinetically favoured over intrastrand secondary structures.

Thus, in a further aspect of the invention the products of nucleic acid amplification are detected using real-time techniques. In one specific embodiment of the invention the real-time technique consists of using any one of the Taqman^{®} system, the Molecular beacons system or the Scorpion probe system.

The QMSP approach may be more sensitive and more specific than conventional PCR and may detect aberrant methylation patterns in human samples with substantial (1:10.000) contamination of normal DNA (12). Moreover, the PCR reaction is amenable to high-throughput techniques allowing the analysis of approximately 400 samples in less then 2 hours without a requirement for gel-electrophoresis.

The steps of the method of the invention for this embodiment may be as follows:
a) treat sample with sodium bisulphite,
b) alkaline hydrolysis to convert unmethylated cytosine residues to uracil,
c) amplify sequences of interest using specific primers designed to distinguish between methylated and unmethylated cytosine residues; and
d) detect the products of amplification via gene specific probe molecules.

However, it is clear that other methods that may be used to determine the methylation status of the panel of genes may be utilised in the method of the invention. Alternative methods of detection of the methylation status of the panel of genes that may be used include mass spectrometry, including MALDI and/or MALDI-TOF mass spectrometry. For example, primers may be included which are designed such that they only direct amplification of methylated sequences. Mass spectrometry may be used in order to detect a label which is only produced/bound to the amplificate if amplification occurs. The method may, alternatively, take advantage of differences in molecular weight of the products following bisulphite treatment depending upon whether the appropriate cytosine residues are methylated or unmethylated.

Also within the contemplation of the present invention is the use of microarray technology (Motorola, Nanogen). With respect to a microarray, multiple suitable CpG island tags may be arrayed as templates on a solid support. These tags represent the sites of potential methylation of interest in diagnosing cervical cancer, potentially in both unmethylated and methylated forms. The solid support may be a microchip for example. Amplicons, such as, for example, at least one of those amplified by the primers comprising, consisting essentially of or consisting of the sequences as set out in SEQ ID NO: 1 and 2, 4 and 5, 7 and 8, 13 and 14, 16 and 17, 19 and 20, 22 and 23, 25 and 26, 28 and 29, 31 and 32, 34 and 35, 37 and 38, 40 and 41, and 43 and 44 with amplicons produced using primers comprising, consisting essentially of or consisting of the sequences of SEQ ID NO: 49 and 50 (as internal reference) and SEQ ID NO: 46 and 47 (as negative control) may be prepared from test samples and also control cells (positive and negative controls). These amplicons may then be used to probe the arrays in order to detect the methylation status of the panel of genes and therefore provide a cervical cancer diagnosis. By determining hybridisation at specific sites the methylation status of the genes of interest in the sample is thus determined.

The read out from the methods is preferably a fluorescent read out, but may comprise, for example, an electrical or radiation read out.

In a preferred embodiment, the methylation status of ESR1 is determined using primers comprising, consisting essentially of, or consisting of the sequence of SEQ ID NO:1 and SEQ ID NO:2 and a probe comprising or consisting essentially of, or consisting of the sequence of SEQ ID NO:3. If, for example, the method also comprises determining the methylation status of DAP-kinase, then primers comprising or consisting essentially of or consisting of the sequence of SEQ ID NO:4 and SEQ ID NO:5 and a probe comprising or consisting essentially of or consisting of the sequence of SEQ ID NO:6 may, in addition, be utilised.

Furthemore, QMSP requires an internal standard gene against which background the methylation of the panel of genes may be measured. In a preferred embodiment of the invention, the internal reference gene used is β-actin. Accordingly, detection of the methylation status of the β-actin gene by QMSP may be carried out using primers and (optionally, depending upon whether MSP or QMSP is being utilised) probe sequences comprising or consisting essentially of or consisting of those as set out in SEQ ID NO's 49 to 51.

In addition analysis of a negative control gene is preferably included. The preferred choice as negative control gene is β-catenin. Detection of the methylation status of the β-catenin gene may be carried out using primers and (optionally, depending upon whether MSP or QMSP is being utilised) probe sequences comprising or consisting essentially of or consisting of those set out in SEQ ID NO: 46 to 48.

In a most preferred embodiment the sample tested is a cervical scraping as this sample type appears to give the most reliable results. However, the invention is not limited to only this sample type. For example, tissue samples such as paraffin embedded tissue samples are also within the scope of the invention.

Any specific subtype of cervical cancer, such as, for example, squamous cell carcinoma (SCC), or adenocarcinoma may be diagnosed utilising the method and kits of the invention.

The method of the invention may additionally comprise the step of obtaining the sample from the subject. For example the method of the invention may include the step of taking a cervical scraping from a subject who is being tested for cervical cancer.

The methods described may be implemented using various kits of the invention. Accordingly, in a further aspect the invention provides a kit for use in a method of detecting susceptibility to, or the incidence of, cervical cancer in a sample (which comprises determining the methylation status of ESR1) comprising:
a) gene specific primers for ESR1 which allow determination of the methylation status of said gene; and
b) means for contacting said primers with said sample.

As for the method, the kit may allow determination of whether a specific gene is hypermethylated at particular cytosine residues.

Such a kit allows the MSP method of detecting susceptibility to, or the incidence of, cervical cancer in a sample to be carried out. In order to carry out the embodiment of the method of the invention wherein QMSP is utilised in order to determine the methylation status of the ESR1 gene, the kit additionally comprises gene specific probes.

Therefore, in a still further aspect of the invention there is provided a kit for use in a method of detecting susceptibility to, or the incidence of, cervical cancer in a sample (which comprises determining the methylation status of ESR1) comprising:
a) gene specific primers and probes for ESR1 which allow determination of the methylation status of said gene; and
b) means for contacting said primers and probes with said sample.

The kits are also useful for detecting susceptibility to, or the incidence of, cervical cancer in a sample by determining the methylation status of a panel of genes including ESR1. In this preferred embodiment the kit comprises:
a) gene specific primers which allow determination of the methylation status of the genes, including ESR1, whose methylation status is linked to the incidence of cervical cancer; and
b) means for contacting said primers and probes with said sample.

If the QMSP method is utilised the kit for diagnosing susceptibility to, or the incidence of, cervical cancer in a sample comprising determining the methylation status of a panel of genes, including ESR1, for which their methylation status is linked to the incidence of cervical cancer preferably comprises:
a) gene specific primers and probes which allow determination of the methylation status of the genes, including ESR1, whose methylation status is linked to the incidence of cervical cancer; and
b) means for contacting said primers and probes with said sample.

The gene specific primers and probes which may be included in the kits of the invention and employed in the methods of the invention are described in more detail below.

However, in a most preferred embodiment the kits according to these aspects of the invention comprises gene specific primers comprising or consisting essentially of or consisting of the sequence of SEQ ID NO: 1 and 2. These primers are specific for determining the methylation status of the ESR1 gene and are suitable for use in both MSP and QMSP methods. The primers will direct amplification only if the selected cytosine residues in the ESR1 gene are methylated.

In a preferred embodiment of the kit suitable for detecting susceptibility to, or the incidence of, cervical cancer in a sample which comprises determining the methylation status of (at least) ESR1 using the QMSP method, the kit comprises a gene specific probe comprising or consisting essentially or consisting of the sequence of SEQ ID NO: 3.

In further embodiments, the kits of the invention may further comprise any, some or all of DNA isolation reagents, enzymes for amplification of DNA, sodium bisulphite and suitable buffers. Such reagents are generally commercially available and suitable reagents and protocols for DNA isolation and amplification etc are described in standard laboratory manuals such as Sambrook et al, "Molecular Cloning: A Laboratory Manual" (2nd.ed.), Vols. 1-3, Cold Spring Harbor Laboratory Press (1989) and F. Ausubel et al, eds., "Current protocols in molecular biology", Green Publishing and Wiley Interscience, New York (1987).

DNA isolation reagents are needed in order to purify DNA from samples, which may be cervical scrapings or tissue samples for example. Such DNA isolation reagents are well known in the art, for example phenolchloroform extraction is a commonly used technique. Kits may include phosphate buffered saline (PBS) for suspending cells and wash buffer (10 mM HEPES-KOH (pH=7.5); 1.5 mM MgCl₂; 10 mM KCl; 1 mM dithiothreitol). DNA may be extracted using standard salt-chloroform techniques and therefore such reagents may be included in the kits of the invention. Ethanol precipitation may be used to obtain high molecular weight DNA, and such reagents used in this technique may be included within the kits of the invention. TE buffer (10 mM Tris; 1 mM EDTA (pH 8.0)) may also be included for dissolving DNA samples. Alternatively, for example, distilled water may be used.

As both the MSP and QMSP techniques are well known in the art suitable buffers and enzymes will be familiar to a person of skill in the art. Primers are designed that amplify the region of the genes that will be affected by sodium bisulphite treatment depending upon the methylation status of the genes. Probes, generally containing a fluorescer and a quencher at opposite ends (e.g. Taqman probes or molecular beacons) are also designed, in the case of the QMSP method, such that they bind in between the primers that amplify the methylated region.

Any suitable fluorophore is included within the scope of the invention. Fluorophores that may possibly be used in the method of the invention include, by way of example, FAM, HEX^{™} , NED^{™}, ROX^{™}, Texas Red^{™} etc. Similarly the kits of the invention are not limited to a single quencher. Quenchers, for example Dabcyl and TAMRA are well known quencher molecules which may be used in the method of the invention.

Reagents may be required for the sodium bisulphite treatment of the extracted DNA. Also required are PCR enzymes, such as Taq polymerase, which may be thermostable, in order to amplify the DNA sequences. As the MSP and QMSP techniques are well known in the art, the reagents necessary for their implementation will also be well known to one of skill in the art. All such reagents are included in the scope of the present invention.

The kits of the invention may also include instructions which describe how to carry out the method of the invention using the kit. The instructions may be included as an insert, possibly provided within the kits packaging, or may be printed on the packaging for example.

In further embodiments the kits of the invention may also contain means for removing cervical cells from a patient for analysis. For example an Ayre's spatula and/or an endocervical brush may be used in order to obtain a cervical sample.

In order to detect the methylation status of the genes of interest, as mentioned above, primers are required that allow amplification of the region affected by sodium bisulphite treatment. A forward and reverse primer will normally be provided. Additionally for the QMSP technique, in order to allow real time detection, a probe, preferably a fluorescent probe, may be required. A list of suitable forward and reverse primers and probes for some of the various genes, including ESR1, which may be included in a panel is given below. Throughout the specification, reference to primer pairs is made firstly to the forward primer and secondly to the reverse primer. It should be noted that the invention is not limited to these specific sequences. Any primers and probes that allow suitable detection of the methylation status of the relevant genes by QMSP or MSP fall within the scope of the invention. Similarly, conservative substitutions in the sequences that do not significantly affect the specificity of binding to the target sequences are within the scope of the invention.

The primers and probes may typically be synthesised nucleic acid sequences. However, the sequences may also incorporate non-natural or derivatised bases, provided specificity of binding to the target sequences is retained. For example, use of phosphorothioate nucleic acids (PNA) may aid with mass spectrometry detection.

All genes referred to herein below may be included in the methods and kits of the invention, in terms of being suitable for having their methylation status determined:
(a) ESR1 methylation specific
   SEQ ID NO:1 5'-GGC GTT CGT TTT GGG ATT G-3'
   SEQ ID NO:2 5'-GCC GAC ACG CGA ACT CTA A-3' 
SEQ ID NO:3 6FAM5'-CGA TAA AAC CGA ACG ACC CGA CGA-
3'TAMRA
(b) DAPK methylation specific
   SEQ ID NO:4 5'-GGA TAG TCG GAT CGA GTT AAC GTC-3'
   SEQ ID NO:5 5'-CCC TCC CAA ACG CCG A-3' 
SEQ ID NO:6     6FAM5'-TTC GGT AAT TCG TAG CGG TAG GGT
                TTG G-3'TAMRA
(c) APC methylation specific,
   SEQ ID NO:7 5'-GAA CCA AAA CGC TCC CCA T-3'
   SEQ ID NO:8 5'-TTA TAT GTC GGT TAC GTG CGT TTA TAT-3' 
SEQ ID NO:9 6FAM5'-CCC GTC GAA AAC CCG CCG ATT A-
3'TAMRA
(d) p16 methylation specific,
   SEQ ID NO:10 5'- TTA TTA GAG GGT GGG GCG GAT CGC-3'
   SEQ ID NO:11 5'-GAC CCC GAA CCG CGA CCG TAA-3' 
SEQ ID NO:12    6FAM5'-AGT AGT ATG GAG TCG GCG GCG GG-
                3'TAMRA
(e) GSTP1 methylation specific
   SEQ ID NO:13 5'-AGT TGC GCG GCG ATT TC-3'
   SEQ ID NO:14 5'-GCC CCA ATA CTA AAT CAC GAC G-3' SEQ ID NO:15   6FAM5'-CGG TCG ACG TTC GGG GTG TAG CG- 3'TAMRA
(f) MGMT methylation specific
   SEQ ID NO:16 5'-CGA ATA TAC TAA AAC AAC CCG CG-3'
   SEQ ID NO:17 5'-GTA TTT TTT CGG GAG CGA GGC-3' 
SEQ ID NO:18   6FAM5'-AAT CCT CGC GAT ACG CAC CGT TTA
               CG-3'TAMRA
(g) TIMP3 methylation specific
   SEQ ID NO:19 5'-GCG TCG GAG GTT AAG GTT GTT-3'
   SEQ ID NO:20 5'-CTC TCC AAA ATT ACC GTA CGC G-3' 
SEQ ID NO:21    6FAM5'-AAC TCG CTC GCC CGC CGA A
                -3'TAMRA
(h) RAR-beta methylation specific 
SEQ ID NO:22    5'-GGG ATT AGA ATT TTT TAT GCG AGT TGT
                -3'
 SEQ ID NO:23 5'-TAC CCC GAC GAT ACC CAA AC-3' 
SEQ ID NO:24    6FAM5'-TGT CGA GAA CGC GAG CGA TTC G-
                3'TAMRA
(i) CALCA methylation specific
   SEQ ID NO:25 5'-GTT TTG GAA GTA TGA GGG TGA CG-3'
   SEQ ID NO:26 5'-TTC CCG CCG CTA TAA ATC G-3' 
SEQ ID NO:27    6FAM5'-ATT CCG CCA ATA CAC AAC AAC CAA
                TAA ACG-3'TAMRA
(j) MLH1 methylation specific 
SEQ ID NO:28 5'- CGT TAT ATA TCG TTC GTA GTA TTC GTG
TTT-3'
 SEQ ID NO:29 5'- CTA TCG CCG CCT CAT CGT-3' 
SEQ ID NO:30 6FAM5'- CGC GAC GTC AAA CGC CAC TAC G-
3'TAMRA
(k) RassfiA methylation specific
   SEQ ID NO:31 5'- GCG TTG AAG TCG GGG TTC -3'
   SEQ ID NO:32 5'- CCC GTA CTT CGC TAA CTT TAA ACG -3' 
SEQ ID NO:33 6FAM5'- ACA AAC GCG AAC CGA ACG AAA
CCA-3'TAMRA
(l) FHIT
   SEQ ID NO:34 5'-GGG CGC GGG TTT GGG TTT TTA C-3'
   SEQ ID NO:35 5'-GAA ACA AAA ACC CAC CGC CCC G-3' 
SEQ ID NO:36   6FAM5'-AAC GAC GCC GAC CCC ACT AAA CTC
C-3'TAMRA.
(m) THBS1
   SEQ ID NO: 37 5'-CGA CGC ACC AAC CTA CCG-3'
   SEQ ID NO: 38 5'-GTT TTG AGT TGG TTT TAC GTT CGT T-3'
   SEQ ID NO: 39 6FAM5'-ACG CCG CGC TCA CCT CCC T-3'TAMRA
(n) CDH1
   SEQ ID NO:40 5'-AAT TTT AGG TTA GAG GGT TAT CGC GT-3'
   SEQ ID NO:41 5'-TCC CC AAA ACG AAA CTA ACG AC-3' 
SEQ ID NO:42 6FAM5'-CGC CCA CCC GAC CTC GCA T-
3'TAMRA.
(o) HIC1
   SEQ ID NO:43 5'-GTT AGG CGG TTA GGG CGT C -3'
   SEQ ID NO:44 5'-CCG AAC GCC TCC ATC GTA T-3' 
SEQ ID NO:45   6FAM5'-CAA CAT CGT CTA CCC AAC ACA CTC
TCC TAC G -3'TAMRA.
(p) β-catenin
   SEQ ID NO:46 5'-GGA AAG GCG CGT CGA GT-3'
   SEQ ID NO:47 5'-TCC CCT ATC CCA AAC CCG-3'
   SEQ ID NO:48 6FAM5'-CGC GCG TTT CCC GAA CCG-3'TAMRA.
(q) β-actin
   SEQ ID NO:49 5'-TGG TGA TGG AGG AGG TTT AGT AAG T-3'
   SEQ ID NO:50 5'-AAC CAA TAA AAC CTA CTC CTC CCT TAA-3'

SEQ ID NO:51   6FAM5'-ACC ACC ACC CAA CAC ACA ATA ACA
               AAC ACA-3'TAMRA.

Thus, in a further aspect the invention provides certain probes and primers that may be used in the methods and kits of the invention. As stated above each primer and probe set, comprising a forward and reverse primer and a fluorescent probe, allows specific amplification of a specific region of a particular gene whose methylation status is linked to cervical cancer. The specific primers and probes are shown in SEQ ID NO's 1-45. Also provided are the primers and probes required for amplification of the internal reference gene β-actin, as shown in SEQ ID NO's 49-51, and the negative control gene, β-catenin as depicted in SEQ ID NO: 46-48.

Thus, in a preferred embodiment, the invention provides a primer comprising or consisting essentially of or consisting of the sequence of SEQ ID NO: 1. The invention also provides a primer comprising or consisting essentially of or consisting of the sequence of SEQ ID NO: 2. Preferably the primers are utilised as a primer pair and so a primer set is provided comprising or consisting essentially of or consisting of the sequences of SEQ ID NO:1 and SEQ ID NO: 2.

In a further preferred embodiment the invention provides a probe molecule comprising or consisting essentially of or consisting of the sequence of SEQ ID NO:3. Such a probe may have attached a suitable molecule to aid detection such as a flurophore, or a flurophore/quencher pair.

FAM is a well known and commercially available fluorophore. The invention is not limited to using this specific fluorophore. Any suitable fluorophore is included within the scope of the invention. Alternative fluorophores that may possibly be used in the method of the invention include, by way of example, HEX^{™}, NED^{™}, ROX^{™}, Texas Red^{™} etc.

Similarly, the invention is not limited to the commercially available quencher TAMRA. Other quenchers are well known in the art, for example Dabcyl is a well known quencher molecule that may be used in the method of the invention.

As described above, a suitable internal standard gene is used with the QMSP technique. In a preferred embodiment, the internal reference gene is β-actin. However, other internal reference genes may be used, as long as the methylation status of these genes is not linked to cervical cancer.

In one embodiment a forward and reverse primer, comprising or consisting essentially of or consisting of the sequence of SEQ ID NO:49 and 50 respectively and a fluorescent probe comprising or consisting of the sequence of SEQ ID NO:51 are used to detect the methylation status of the relevant region of the β-actin gene, as shown below. It will be appreciated, however, that any primers and probes that allow detection of the relevant region of the β-actin gene by QMSP depending upon whether the appropriate cytosine residues are methylated or not will fall within the scope of the invention.
(q) β-actin
   SEQ ID NO:49 5'-TGG TGA TGG AGG AGG TTT AGT AAG T-3'
   SEQ ID NO:50 5'-AAC CAA TAA AAC CTA CTC CTC CCT TAA-3'

SEQ ID NO:51   6FAM5'-ACC ACC ACC CAA CAC ACA ATA ACA
AAC ACA-3'TAMRA.

Preferably the results of QMSP analysis are expressed as ratio's between two absolute measurements:
1) cycle number of crossing threshold for internal reference
2) cycle number of gene of interest

This ratio may require multiplication by a certain number in order to achieve a result that is more easily presented. The threshold is set to the geometrical phase of the amplification above the background. However the invention is not limited to this specific threshold. Any threshold may be used providing it gives sensitive and specific results.

In a most preferred embodiment the ratio's obtained will be compared against a control ratio using the well known Mann Whitney U test in order to achieve a result as either positive or negative for susceptibility to, or the incidence of, cervical cancer. However, the skilled person will realise that other forms of statistical analysis may be used in order to achieve a diagnostic result, and the invention is not limited to only this test. An association at a level of p ≤0.05 (or even below this) is preferred, but other significance levels are within the scope of the invention.

Similarly, the level that the sample has to reach above control in order to be classified positive for susceptibility to, or the incidence of, cervical cancer may be balanced in order to achieve maximal sensitivity for the test, whilst retaining selectivity.

It is known that methylation of genes, particularly in the CpG island regions, may lead to cancer, such as cervical cancer. CpG island hypermethylation is often associated with a transcriptional block and subsequent loss of translation of the relevant protein. In many cases an important tumour suppressor protein is lost.

Therefore, in a further aspect of the invention there is provided a method of detecting susceptibility to, or the incidence of, cervical cancer in a sample comprising detecting/determining the RNA expression levels of ESR1. The level of ESR1 (m)RNA will be decreased in a cancer sample, relative to a control, because (hyper)methylation of the gene whose methylation status is linked to cancer leads to a decrease in transcription of the gene.

As has already been described for the method according to the first aspect of the invention, ESR1 may form part of a panel of genes which are assessed in order to detect susceptibility to, or the incidence of, cervical cancer in a sample. In this aspect of the invention, the RNA expression level is assessed in order to diagnose susceptibility to, or the incidence of, cervical cancer in a sample. There are a number of genes whose methylation status is known to be linked to the incidence of cervical cancer and whose resultant reduction in RNA expression levels may thus be utilised in order to diagnose cervical cancer.

Therefore, in one embodiment of the invention, the panel of genes whose RNA levels are determined in order to detect susceptibility to, or the incidence of, cervical cancer also comprises, in addition to ESR1, at least one of the following genes: DAP-kinase, APC, TIMP-3, RAR-beta, CALCA, TSLC1, TIMP-2, DcR1, DcR2, BRCA1, p15, Rassf1A, MLH1, MGMT. Most preferably, the panel will comprise at least detecting the RNA levels of DAP-kinase in addition to those of ESR1.

In an additional aspect of the invention, the method of detecting susceptibility to, or the incidence of, cervical cancer comprises detecting the RNA levels of a total of three genes. At least one of these will be ESR1. The other two genes can be selected from any gene whose methylation status is linked to susceptibility to, or the incidence of, cervical cancer and whose RNA levels are decreased in a cervical cancer sample as a result of this methylation. For example, the remaining two genes may be selected from the following genes: DAP-kinase, APC, TIMP-3, RAR-beta, CALCA, TSLC1, TIMP-2, DcR1, DcR2, BRCA1, p15, Rassf1A, MLH1, MGMT. Preferably, the remaining two genes whose RNA expression levels are determined in the sample are selected from DAP-Kinase, APC, TIMP-3, CALCA and RAR-beta.

In a further embodiment, the method of detecting susceptibility to, or the incidence of, cervical cancer comprises detecting the RNA expression levels of a total of four genes. At least one of these will be ESR1. The remaining three genes may be selected from any gene whose methylation status is linked to cervical cancer, resulting in down-regulation of RNA expression. For example, the remaining three genes may be selected from the following genes: DAP-kinase, APC, TIMP-3, RAR-beta, CALCA, TSLC1, TIMP-2, DcR1, DcR2, BRCA1, p15, Rassf1A, MLH1, MGMT. Preferably, the remaining three genes whose RNA expression levels are determined in the sample are selected from DAP-Kinase, APC, TIMP-3, CALCA and RAR-beta.

In a still further embodiment, the method of detecting susceptibility to, or the incidence of, cervical cancer comprises detecting the RNA expression levels of a total of five genes. At least one of these will be ESR1. The remaining four genes may be selected from any gene whose methylation status is linked to susceptibility to, or the incidence of, cervical cancer, leading to down-regulation of RNA expression. For example, the remaining four genes may be selected from the following genes: DAP-kinase, APC, TIMP-3, RAR-beta, CALCA, TSLC1, TIMP-2, DcR1, DcR2, BRCA1, p15, Rassf1A, MLH1, MGMT. Preferably, the remaining four genes whose RNA expression levels are determined in the sample are selected from DAP-Kinase, APC, TIMP-3, CALCA and RAR-beta.

The decreased RNA levels may be detected using any suitable technique. There are a large number of techniques which are well known in the art and rely on suitable isolation of the RNA. For example, reverse-transriptase-PCR (RT-PCR) is a well known technique for determining RNA expression. RT-PCR relies upon an enzyme called reverse transcriptase which may use single stranded RNA as a template for production of double stranded cDNA (complementary DNA) which may subsequently be amplified using the polymerase chain reaction (PCR).

The subsequent PCR step, following reverse transcription may be real-time quantitative PCR. This may require, for example, fluorescent probes (such as Taqman probes or molecular beacons) in addition to suitable gene specific PCR primers, which are specific for the genes whose methylation status is linked to susceptibility to, or the incidence of, cervical cancer. Examples of such gene specific probes are represented in SEQ ID NO's 3, 6, 9, 15, 18, 21, 24, 27, 30, 33, 39, 42 and 45. The primers described above may prove suitable for amplifying cDNA.

Another technique that may be used is microarray technology. If suitable tags, representing the genes whose methylation status is linked to susceptibility to, or the incidence of, cervical cancer are attached to a solid support, probes that detect expression of the panel of genes whose hypermethylation is linked to susceptibility to, or the incidence of, cervical cancer may be used in order to compare expression of the various RNA molecules in the test sample as compared to control samples. The solid support may be in the form of a microchip (Motorola, Nanogen). Other techniques for RNA detection that may be used in this aspect of the invention include mass spectrometry, including MALDI-TOF mass spectrometry.

The read out from the various techniques may be a fluorescent read out, or alternatively detection may be through radiolabelling or an electrical read-out.

It is preferred that this method of the invention allows at least 80% detection of susceptibility to, or the incidence of, cervical cancer, more preferably at least 85% detection of susceptibility to, or the incidence of, cervical cancer and even more preferably at least 90% detection of susceptibility to, or the incidence of, cervical cancer, most preferably at least 95% detection of susceptibility to, or the incidence of, cervical cancer. An especially preferred method of the invention would utilise a panel of no more than five genes, preferably no more than four genes and even more preferably three genes which, when analysed together, give at least (about) 90% detection of susceptibility to, or the incidence of, cervical cancer.

The methods according to this aspect of the invention may take place in a multiplexing context as described in more detail above, such that the expression of all genes of interest at the RNA level is assessed in a single experiment and this is preferred although not limiting with respect to the invention.

In a further aspect of the invention kits allowing methods of detecting susceptibility to, or the incidence of, cervical cancer in a sample comprising detecting/determining the RNA expression levels of ESR1 are provided. Kits for assessing a panel of genes (including ESR1) whose methylation status is linked to susceptibility to, or the incidence of, cervical cancer and for which methylation of the gene leads to a decrease in RNA expression levels are also provided. The panel of genes comprise the panels discussed with respect to the methods of the invention.

Accordingly, included in the kits are sequence specific primers that prime reverse transcription for the genes whose methylation status is linked to cervical cancer. Additionally sequence specific primers are also required for the amplification stages. Primers for the amplification stages may comprise primers such as those shown in SEQ ID NO: 1 and 2, 4 and 5, 7 and 8, 13 and 14, 16 and 17, 19 and 20, 22 and 23, 25 and 26, 28 and 29, 31 and 32, 34 and 35, 37 and 38, 40 and 41, and 43 and 44.

However, the invention (with the exception of ESR1) is not limited to these specific genes for detection of susceptibility to, or the incidence of, cervical cancer and so many other primers may be used to detect reduced expression of other genes whose methylation status is linked to cervical cancer.

A kit for diagnosing susceptibility to, or the incidence of, cervical cancer in a sample comprising determining the RNA levels of those genes whose methylation status is linked to cervical cancer is provided comprising:
a) gene specific primers for the genes whose methylation status is linked to the incidence of cervical cancer for reverse transcription and for second strand synthesis and further amplification; and
b) means for contacting said primers with said sample.

In one embodiment the kits further contain reagents necessary for RT-PCR. The RT-PCR protocol can be found in standard laboratory handbooks and suitable reagents are commercially available. Such components may include, by way of example, a reverse transcriptase such as AMV reverse transcriptase for first strand cDNA synthesis. For second strand cDNA synthesis and subsequent amplification a DNA polymerase, such as Tfl DNA polymerase would be required. It is important in RT-PCR reactions that conditions are kept ribonuclease (RNase) free. Therefore an RNase inhibitor may be included in the kit to prevent degradation of the RNA samples.

A kit for use with a microarray method is also provided. Such a kit may include, by way of example, primers used to amplify the genes, including ESR1, for which (hyper)methylation affects their expression levels. Preferably, the kit may provide a solid support with the tags for the appropriate genes pre-loaded and attached. The kit may also provide suitable labels, such that RNA binding to the tags may be detected. The kit may also comprise means for contacting the tags with the sample. The kit may optionally further comprise RNA isolation reagents, appropriate buffers and an array containing suitable tags attached to a support. The array may be in the form of a microchip.

A kit for use in a method of detecting susceptibility to, or the incidence of, cervical cancer wherein RNA expression levels are detected by mass spectrometry may include components such as gene specific primers for the genes whose methylation status is linked to susceptibility to, or the incidence of, cervical cancer for reverse transcription and for second strand synthesis and further amplification and means for contacting said primers with said sample. The kit may further include RNA isolation reagents and appropriate buffers. Such reagents and buffers are well known in the art.

The invention will be further understood with reference to the following examples, together with the accompanying tables and figures in which:
Table 1 shows the primer pairs, amplicon sizes and Genbank accession numbers for the genes assessed in the experiments for assessment of their importance in cervical cancer.
Table 2 compares methylation of specific genes in cervical cancer scrapings (both squamous cell carcinoma and adenocarcinoma) as compared to control samples.
Table 3 shows the levels of methylation of specific genes in squamous cell carcinoma samples and adenocarcinoma samples.
Table 4 compares diagnosis by studying morphology against results achieved by measuring methylation of the panel of genes for control samples.
Table 5 compares diagnosis by studying morphology against results achieved by measuring methylation of the panel of genes for cervical cancer scraping samples.

### Patients and methods

### Patients

Cervical scrapings were collected from 35 cervical cancer patients and 20 controls.

All patients referred between March 2001 and September 2003 because of cervical cancer were asked to participate in our study during their initial visit to the outpatient clinic of the Groningen University Medical Centre. Gynaecological examination under general anaesthesia was performed in all cervical cancer patients for staging in accordance with the FIGO criteria (0). All cervical scrapings were collected during the initial visit or before bimanual examination under general anaesthesia. As controls we asked 20 women without a history of abnormal papsmears to participate. These women were undergoing a hysterectomy for benign reasons in the same period of time. Cervical scrapings were collected during surgery. All patients gave written consent.

### Sample collection

The cervical scrapings were collected using an Ayre's spatula and an endocervical brush. The spatula and brush with the collected cells were then suspended in 5 ml of phosphate buffered saline (PBS:6.4 mM Na2HPO4; 1.5 mM KH2PO4; 0.14 M NaCl; 2.7 mM KCl (pH 7.2)) and kept on ice until further processing. Of this cell suspension 1 ml was used for cytomorphological examination and 4 ml was centrifuged, washed and snap-frozen in liquid nitrogen as described previously(21). DNA was extracted using standard salt-chloroform extraction and ethanol precipitation for high molecular DNA and dissolved in 250 µl TE-4 buffer (10 mM Tris; 1mM EDTA (pH 8.0).

### Real-time quantitative Methylation Specific PCR

QMSP was performed after bisulfite treatment on denatured genomic DNA (11) as previously reported for APC and GSTP1(14,15). As internal reference gene the β-actin gene was chosen. Primer pairs, amplicon size and Genbank accession number are listed in table 1. The basis of primer design has been previously described(14,19). Amplifications were carried out in 384-well plates. As positive controls serial dilutions of in vitro CpG methylated DNA with Sss I (CpG) methylase (New England Biolabs. Inc., Beverly, MA) were used by which a calibration curve was constructed for each plate. The calibration curve was used to set a plate specific threshold for positivity and to determine DNA equivalents for the results obtained. Multiple water blanks were included as negative controls. Dilution experiments showed linearity of amplification down to a dilution of 1:10,000 for methylated promoter DNA, as well as for unmethylated β-actin DNA. All samples were analyzed in triplicate. For quality control all amplification curves were visualized and scored without knowledge of the clinical data. Per analysis the methylation result was considered positive when the QMSP amplification curve crossed the set threshold before 50 cycles. However, amplification above threshold without an exponential curve was considered to be the result of stochastic amplification and the results of such a single analysis was therefore disregarded. For statistical analysis, for every single gene only a sample with sufficient DNA input (at least 225 pg β-actin DNA because in samples with DNA input below 225 pg stochastic amplification was too frequent) that was positive at least twice after multiple analyses was considered to be hypermethylation positive. A sample with sufficient DNA input that was negative at least twice and at most one time positive after multiple analyses was considered to be negative.

### Statistical analysis

QMSP values were adjusted for DNA input by expressing results as ratios between two absolute measurements ((average DNA quantity of methylated gene of interest / average DNA quantity for internal reference gene β-actin) x 10000) (more detailed information was described previously(12,14). Differences in the heights of ratios between cancer patients and controls were tested with the Mann-Whitney U test for all genes. Associations between numerical parameters were analyzed with the chi-square test with Yates' correction for small numbers. To evaluate the clinical value of QMSP in cervical scrapings 'screen-positive' cut-off values were chosen above the highest ratio observed in controls for all genes separately.

Observed differences were considered to be significant when associated with p ≤ 0.05. All analyzes were carried out using the SPSS software package (SPSS 11.5, Chicago, IL, USA).

### Results

### Patients

Of the 35 cervical cancer patients whose cervical scapings were used in this study, 10 were diagnosed with adenocarcinoma (29%) and 25 with squamous cell carcinoma (71%). Of all cervical cancer patients three were diagnosed with FIGO stage IA (9%), 13 had FIGO stage IB (37%), 4 had stage IIA(11%), 11 had stage IIB(31%), 2 had FIGO stage IIIA (6%) and 2 were stage IV (6%). The mean age of the cervical cancer patients was 46 yrs. (standard deviation 14.6) and for the controls 54 yrs. (standard deviation 12.9). Seven scrapings could not be used for QMSP because of poor DNA quality (5 squamous cell carcinoma samples and 2 adenocarcinoma samples). In all control samples DNA was available for analysis.

### QMSP as a diagnostic tool

In our previous study we showed that the hypermethylation status of the underlying lesion was perfectly reflected in the cervical scraping. Therefore, we tested QMSP only on cervical scrapings of cervical cancer patients and controls. DNA quality was sufficient to perform QMSP on 48 cervical scrapings (20 SCC, 8 adenocarcinomas and 20 controls). Table 1 shows the presence of hypermethylation in cervical cancers and controls. Methylated ESR1, DAPK, APC, TIMP3 and RAR-β were more detected in scrapings of cervical cancer patients than in controls. And when artificial cut-off points (above the highest control) were chosen CALCA was also more methylated in cervical cancer patients compared to controls (see table 2). Overall 24 of 28 (86%) cervical cancer patients were positive (above the highest control) for at least one gene, 16 of 20 (80%) SCC patients and 8 of 8 (100%) adenocarcinoma patients.

Table 3 shows the presence of hypermethylation in SCC and adenocarcinoma patients. There was no difference between methylation of SCC and adenocarcinomas. However, when artificial cut-offs were defined above the highest control methylation of APC, TIMP3 and RASSF1A was more detected in adenocarcinoma patients compared to SCC patients. None of the tested genes were more methylated in SCC patients compared to adenocarcinoma patients.

### QMSP versus morphological assessment

Table 4 and 5 show the relation of morphological assessment in relation to presence of methylation above the highest control in controls and cervical cancer patients, respectively. Hypermethylation status was defined as above highest control, therefore hypermethylation status of all controls were negative.

In controls morphological analysis could be performed on 19 samples, while from all controls (n=20) DNA input was sufficient to perform QMSP analysis and by definition these were all negative. Although the epithelium was histologically diagnosed as normal 3 scrapings were classified as borderline dysplastic. From the 30 cervical cancer scrapings 29 could be morphologically analyzed. 3 of 29 were classified as no or borderline dysplasia, while DNA input was sufficient in 2 scrapings and even 1 was hypermethylation positive. 2 of 29 were inadequate to morphological diagnosis, of these 2 scrapings 1 had sufficient DNA input and was also hypermethylation positive. Specificity and sensitivity for QMSP and morphology were equivalent to each other (QMSP: specificity 100% (20 of 20) and sensitivity 80% (24 of 30) and morphology: specificity 84% (16 of 19) and sensitivity 83% (24 of 29)).

**Table 1.**

| Gene | Forward 5'-3' | Probe 6FAM 5'-3'TAMRA | Reverse 5'-3' | Genbank Acc. Number | Amplicon size | Ref |
|---|---|---|---|---|---|---|
| ACTB | TGGTGATGGAGGAGGT TTAGTAAGT | ACCACCACCCAACACAC AATAACAAACACA | AACCAATAAAACCT ACTCCTCCCTTAA | Y00474 | 133 bp; 390-522 | (14) |
| APC | GAACCAAAACGCTCCC CAT | CCCGTCGAAAACCCGCC GATTA (antisense) | TTATATGTCGGTACGTGCGTTTATAT | U02509 | 74 bp; 761-834 | (14) |
| BCATE NIN | GGAAAGGCGCGTCGAG T | CGCGCGTTTCCCGAACC G | TCCCCTATCCCAAACCCG | X89448 | 82 bp; 583-664 | (25) |
| CALCA | GTTTTGGAAGTATGAG GGTGACG | ATTCCGCCAATACACAA CAACCAATAAACG | TTCCCGCCGCTATAAATCG | X15943 | 101 bp; 1806 | (25) |
| CDH1 | AATTTTAGGTTAGAGG GTTATCGCGT | CGCCCACCCGACCTCGC AT (ANTISENSE) | TCCCCAAAACGAAA CTAACGAC | L34545 | 70 bp; 842-911 | (25) |
| DAPK | GGATAGTCGGATCGAG TTAACGTC | TTCGGTAATTCGTAGCG GTAGGGTTTGG | CCCTCCCAAACGCCGA | X76104 | 98 bp; 5-102 | (26) |
| ESR1 | GGCGTTCGTTTTGGGA TTG | CGATAAAACCGAACGAC CCGACGA | GCCGACACGCGAAC TCTAA | X62462 | 101 bp; 2784-2884 | (25) |
| FHIT (Jero nimo & Hoque ) | GGGCGCGGGTTTGGGT TTTTAC | AACGACGCCGACCCCAC TAAACTCC (antisense) | GAAACAAAAACCCACCGCCCCG | U76263 | 91 bp; 192-293 | |
| HIC1 | GTTAGGCGGTTAGGGC GTC | CAACATCGTCTACCCAA CACACTCTCCTACG (antisense) | CCGAACGCCTCCATCGTAT | L41919 | 101 bp; 562-662 | (25) |
| MLH1 | CGTTATATATCGTTCG TAGTATTCGTGTTT | CGCGACGTCAAACGCCA CTACG | CTATCGCCGCCTCATCGT | U26559 | 88 bp; 254-341 | (25) |
| RAR-b2 | GGGATTAGAATTTTTT ATGCGAGTTGT | TGTCGAGAACGCGAGCG ATTCG | TACCCCGACGATACCCAAAC | NM_0009 65 | 92 bp; 63-154 | |
| Rassf 1A | GCGTTGAAGTCGGGGT TC | ACAAACGCGAACCGAAC GAAACCA | CCCGTACTTCGCTAACTTTAAACG | NM_0071 82 ? | 75 bp; 45-119 | (27) |
| THBS1 | CGACGCACCAACCTAC CG | ACGCCGCGCTCACCTCC CT | GTTTTGAGTTGGTTTTACGTTCGTT | J04835 | 75 bp; 1642-1716 | (25) |
| TIMP3 | GCGTCGGAGGTTAAGG TTGTT | AACTCGCTCGCCCGCCG AA (antisense) | CTCTCCAAAATTACCGTACGCG | U33110 | 93 bp; 1051-1143 | (25) |

**Table 2: Diagnostic use of QMSP in scrapings**

| Genes | CC (n=28) | controls (n=20) | p-value | CC* | p-value |
|---|---|---|---|---|---|
| *ESR1* | 18 (64%) | 1 (5%) | <0.001 | 8 (29%) | 0.009 |
| *DAPK* | 17 (61%) | 3 (15%) | 0.002 | 11 (39%) | 0.001 |
| *APC* | 18 (64%) | 5 (25%) | 0.007 | 5 (28%) | 0.046 |
| *TIMP3* | 10 (36%) | 2 (10%) | 0.043 | 6 (21%) | 0.027 |
| *RAR-*β | 5 (18%) | 0 (0%) | 0.046 | 5 (28%) | 0.046 |
| *FHIT* | 28 (100%) | 18 (90%) | 0.09 | 0 | |
| *CALCA* | 28 (100%) | 19 (95%) | 0.23 | 13 (46%) | <0.001 |
| *MLH1* | 3 (11%) | 1 (5%) | 0.48 | 3 (11%) | 0.13 |
| *RASSF1A* | 8 (29%) | 4 (20%) | 0.5 | 2 (7%) | 0.22 |
| *CDH1* | 21 (75%) | 15 (75%) | 1.00 | 0 | |
| *HIC1* | 28 (100%) | 20 (100%) | | 1 (4%) | 0.39 |
| β-*Catenin* | 0 (0%) | 0 (0%) | | | |

| | | | | | |
|---|---|---|---|---|---|
| *: number of CC positive above highest control. | | | | | |

**Table 3: Diagnostic use of QMSP in cervical cancer scrapings**

| genes | sq. (n=20) | adenoc. (n=8) | p-value | sq.* | adenoc.* | p-value |
|---|---|---|---|---|---|---|
| *ESR1* | 12 (60%) | 6 (75%) | 0.45 | 4 (20%) | 4 (50%) | 0.11 |
| *DAPK* | 14 (70%) | 3 (38%) | 0.11 | 9 (45%) | 2 (25%) | 0.33 |
| *APC* | 12 (60%) | 6 (75%) | 0.45 | 1 (5%) | 4 (50%) | 0.005 |
| *TIMP3* | 5 (25%) | 5 (63%) | 0.061 | 1 (5%) | 6 (63%) | 0.001 |
| *RAR-*β | 3 (15%) | 2 (25%) | 0.53 | 3 (15%) | 2 (25%) | 0.53 |
| *FHIT* | 20 (100%) | 8 (100%) | | 0 (0%) | 0 (0%) | |
| *CALCA* | 20 (100%) | 8 (100%) | | 10 (50%) | 3 (38%) | 0.55 |
| *MLH1* | 1 (5%) | 2 (25%) | 0.12 | 1 (5%) | 2 (25%) | 0.12 |
| *RASSF1A* | 5 (25%) | 3 (38%) | 0.51 | 0 (0%) | 2 (25%) | 0.02 |
| *CDH1* | 15 (75%) | 6 (75%) | 1.00 | 0 (0%) | 0 (0%) | |
| *HIC1* | 20 (100%) | 8 (100%) | | 1 (5%) | 0 (0%) | 0.52 |
| β-*Catenin* | 0 (0%) | 0 (0%) | | | | |

| | | | | | | |
|---|---|---|---|---|---|---|
| *: number of CC positive above highest control. | | | | | | |

**Table 4: Relation between morphological diagnosis and hypermethylation status in controls**

| Morphology | n | Sufficient DNA | Hypermeth. status |
|---|---|---|---|
| No dysplasia | 16 | 16 | 0 |
| Borderline dysplasia | 3 | 3 | 0 |
| Not assessed | 1 | 1 | 0 |
| Total | 20 | 20 | 0 |

**Table 5: Relation between morphological diagnosis and hypermethylation status in cervical cancers**

| Morphology | n | Sufficient DNA | Hypermeth. status |
|---|---|---|---|
| No dysplasia | 1 | 1 | 1 |
| Borderline dysplasia | 2 | 1 | 0 |
| Severe dysplasia/CIS | 6 | 6 | 3 |
| Cell cancer | 18 | 18 | 18 |
| Inadequate | 2 | 1 | 1 |
| Not assessed | 1 | 1 | 1 |
| total | 30 | 28 | 24 |

Borderline dysplasia = PAP II en PAP IIIA, lichte dyspl severe dysplasia = PAP IIIA matige dyspl, PAP IIIB en PAP IV

### Reference List

All references are hereby incorporated into the present disclosure in their entirety.
(0) Petterson, F. (Ed.) Annual report on the results of treatment in gynaecological cancer, FIGO, Stockholm, Vol 20 (1988).
(1) Cervical cancer. NIH Consens Statement 1996; 14 (1) :1-38.
(2) Zur Hausen H. Papillomavirus infections--a major cause of human cancers. Biochimica et Biophysica Acta 1996; 1288(2):F55-F78..
(3) Woodman CB, Collins S, Winter H, Bailey A, Ellis J, Prior P et al. Natural history of cervical human papillomavirus infection in young women: a longitudinal cohort study. Lancet 2001; 357(9271):1831-1836.
(4) Herbert A. Is cervical screening working? A cytopathologist's view from the United Kingdom. Hum Pathol 1997; 28(2):120-126.
(5) Robertson JH, Woodend B. Negative cytology preceding cervical cancer: causes and prevention. J Clin Pathol 1993; 46(8):700-702.
(6) Koss LG. Cervical (Pap) smear. New directions. Cancer 1993; 71(4 Suppl): 1406-1412.
(7) Bovicelli A, Bristow RE, Montz FJ. HPV testing: where are we now? Anticancer Res 2000; 20(6C):4673-4680.
(8) Sherman ME, Schiffman M, Cox JT. Effects of age and human papilloma viral load on colposcopy triage: data from the randomized Atypical Squamous Cells of Undetermined Significance/Low-Grade Squamous Intraepithelial Lesion Triage Study (ALTS). J Natl Cancer Inst 2002; 94(2):102-107.
(9) Kulasingam SL, Hughes JP, Kiviat NB, Mao C, Weiss NS, Kuypers JM et al. Evaluation of human papillomavirus testing in primary screening for cervical abnormalities: comparison of sensitivity, specificity, and frequency of referral. JAMA 2002; 288(14):1749-1757.
(10) Baylin SB, Esteller M, Rountree MR, Bachman KE, Schuebel K, Herman JG. Aberrant patterns of DNA methylation, chromatin formation and gene expression in cancer. Hum Mol Genet 2001; 10(7):687-692.
(11) Herman JG, Graff JR, Myohanen S, Nelkin BD, Baylin SB. Methylation-specific PCR: a novel PCR assay for methylation status of CpG islands. Proc Natl Acad Sci U S A 1996; 93(18):9821-9826.
(12) Eads CA, Danenberg KD, Kawakami K, Saltz LB, Blake C, Shibata D et al. MethyLight: a high-throughput assay to measure DNA methylation. Nucleic Acids Res 2000; 28(8):E32.
(13) Wisman GB, Hollema H, de Jong S, Ter Schegget J, Tjong AHS, Ruiters MH et al. Telomerase activity as a biomarker for (pre)neoplastic cervical disease in scrapings and frozen sections from patients with abnormal cervical smear. J Clin Oncol 1998; 16(6):2238-2245.
(14) Usadel H, Brabender J, Danenberg KD, Jeronimo C, Harden S, Engles J et al. Quantitative adenomatous polyposis coli promoter methylation analysis in tumor tissue, serum, and plasma DNA of patients with lung cancer. Cancer Res 2002; 62(2):371-375.
(15) Jeronimo C, Usadel H, Henrique R, Oliveira J, Lopes C, Nelson WG et al. Quantitation of GSTP1 methylation in non-neoplastic prostatic tissue and organ-confined prostate adenocarcinoma. J Natl Cancer Inst 2001; 93(22):1747-1752.
(16) Hanselaar AG. kwaliteit van cytopathologisch onderzoek in het herziene bevolkingsonderzoek naar baarmoederhalskanker. Nederlands Tijdschrift voor Obstetrie en Gynaecologie 1996; 109:207-210.
(17) Dong SM, Kim HS, Rha SH, Sidransky D. Promoter hypermethylation of multiple genes in carcinoma of the uterine cervix. Clin Cancer Res 2001; 7(7):1982-1986.
(18) Virmani AK, Muller C, Rathi A, Zoechbauer-Mueller S, Mathis M, Gazdar AF. Aberrant methylation during cervical carcinogenesis. Clin Cancer Res 2001; 7(3):584-589.
(19) Sanchez-Cespedes M, Esteller M, Wu L, Nawroz-Danish H, Yoo GH, Koch WM et al. Gene promoter hypermethylation in tumors and serum of head and neck cancer patients. Cancer Res 2000; 60(4):892-895.
(20) Gerson SL. Clinical relevance of MGMT in the treatment of cancer. J Clin Oncol 2002; 20(9):2388-2399.
(21) McCarver DG, Hines RN. The ontogeny of human drug-metabolizing enzymes: phase II conjugation enzymes and regulatory mechanisms. J Pharmacol Exp Ther 2002; 300(2):361-366.
(22) Inbal B, Bialik S, Sabanay I, Shani G, Kimchi A. DAP kinase and DRP-1 mediate membrane blebbing and the formation of autophagic vesicles during programmed cell death. J Cell Biol 2002; 157(3):455-468.
(23) Lundberg AS, Hahn WC, Gupta P, Weinberg RA. Genes involved in senescence and immortalization. Curr Opin Cell Biol 2000; 12(6):705-709.
(24) Sieber OM, Tomlinson IP, Lamlum H. The adenomatous polyposis coli (APC) tumour suppressor--genetics, function and disease. Mol Med Today 2000; 6(12):462-469.
(25) Eads CA, Lord RV, Wickramasinghe K, et al. Epigenetic patterns in the progression of esophageal adenocarcinoma. Cancer Res 2001;61:3410-8.
(26) Harden SV, Tokumaru Y, Westra WH, et al. Gene promoter hypermethylation in tumors and lymph nodes of stage I lung cancer patients. Clin Cancer Res 2003;9:1370-5.
(27) Lehmann U, Glockner S, Kleeberger W, von Wasielewski HF, Kreipe H. Detection of gene amplification in archival breast cancer specimens by laser-assisted microdissection and quantitative real-time polymerase chain reaction. Am J Pathol 2000;156:1855-64.
(28) Reesink-Peters N, Wisman GBA, Jéronimo C, et al. Detecting cervical cancer by quantitative promotor hypermethylation assay on cervical scrapings: A feasibility study. Mol.Cancer Res. 2004. May; 2 (5) : 289-95

### Clauses

1. A method of detecting susceptibility to, or the incidence of, cervical cancer in a sample comprising determining the methylation status of ESR1.
2. The method according to clause 1 comprising determining whether the ESR1 gene is hypermethylated.
3. The method according to clause 1 or clause 2 wherein ESR1 forms one of a panel of genes whose methylation status is linked to the incidence of cervical cancer.
4. The method according to clause 3 wherein the panel of genes also comprises at least one of the following genes: DAP-kinase, APC, TIMP-3, RAR-beta, CALCA, TSLC1, TIMP-2, DcR1, DcR2, BRCA1, p15, Rassf1A, MLH1, MGMT.
5. The method according to clause 4 wherein the panel of genes also comprises DAP-kinase.
6. The method according to any one of clauses 1 to 5 wherein the method comprises determining the methylation status of a total of three genes.
7. The method according to clause 6 wherein the three genes comprise ESR1 and two genes selected from DAPK, APC, TIMP-3, CALCA and RAR-beta.
8. The method according to any one of clauses 1 to 5 wherein the method comprises determining the methylation status of a total of four genes.
9. The method according to clause 8 wherein the four genes comprise ESR1 and three genes selected from DAPK, APC, TIMP 3, CALCA and RAR-beta.
10. The method according to any one of clauses 1 to 5 wherein the method comprises determining the methylation status of a total of five genes.
11. The method according to clause 10 wherein the five genes comprise ESR1 and four genes selected from DAPK, APC, TIMP-3, CALCA and RAR-beta.
12. The method according to any one of clauses 1 to 11 wherein the method allows at least 90% detection of cervical cancer.
13. The method according to any one of clauses 1 to 12 wherein the methylation status of the gene(s) is determined using methylation specific PCR (MSP).
14. The method according to clause 13 wherein the methylation status of ESR1 is determined using primers comprising the sequence of SEQ ID NO:1 and SEQ ID NO:2.
15. The method according to clause 13 wherein the methylation status of ESR1 is determined using primers consisting essentially of the sequence of SEQ ID NO:1 and SEQ ID NO:2.
16. The method according to any one of clauses 1 to 12 wherein the methylation status of the gene(s) is determined using quantitative methylation specific PCR (QMSP).
17. The method according to clause 16 wherein the methylation status of ESR1 is determined using primers comprising the nucleotide sequence of SEQ ID NO:1 and SEQ ID NO:2 and a probe comprising the sequence of SEQ ID NO:3.
18. The method according to clause 16 wherein the methylation status of ESR1 is determined using primers consisting essentially of the nucleotide sequence of SEQ ID NO:1 and SEQ ID NO:2 and a probe consisting essentially of the sequence of SEQ ID NO: 3.
19. The method according to any of the preceding clauses wherein the sample is a cervical scraping.
20. A kit for use in the method of any one of the proceeding claims comprising; a) gene specific primers for the gene(s) whose methylation status is linked to the incidence of cervical cancer; and b) means for contacting said primers with said sample.
21. The kit according to claim 20 further comprising gene specific probes for the gene(s) whose methylation status is linked to the incidence of cervical cancer.
22. The kit according to clause 20 or 21 wherein the gene specific primers comprise those comprising the sequence of SEQ ID NO:1 and SEQ ID NO:2.
23. The kit according to clause 21 wherein the gene specific probe comprises that comprising the sequence of SEQ ID NO:3.
24. The kit according to clause 20 or 21 wherein the gene specific primers comprise those consisting essentially of the sequence of SEQ ID NO:1 and SEQ ID NO:2.
25. The kit according to clause 21 wherein the gene specific probe comprises that consisting essentially of the sequence of SEQ ID NO:3.
26. The kit according to any one of clauses 20 to 25 further comprising DNA isolation reagents.
27. The kit according to any one of clauses 20 to 26 further comprising enzymes for amplification of DNA.
28. The kit according to any one of clauses 20 to 27 further comprising sodium bisulphite.
29. The kit according to any one of clauses 20 to 28 further comprising suitable buffers.

## Claims

1. A method of detecting susceptibility to, or the incidence of, cervical cancer in a sample comprising determining the RNA expression levels of ESR1.

2. The method according to claim 1 or wherein ESR1 forms one of a panel of genes whose RNA expression level is linked to the incidence of cervical cancer.

3. The method according to claim 2 wherein the panel of genes also comprises at least one of the following genes: DAP-kinase, APC, TIMP-3, RAR-beta, CALCA, TSLC1, TIMP-2, DcR1, DcR2, BRCA1, p15, Rassf1A, MLH1, MGMT.

4. The method according to claim 3 wherein the panel of genes also comprises DAP-kinase.

5. The method according to any one of claims 1 to 4 wherein the method comprises determining the RNA expression level of a total of three genes.

6. The method according to claim 5 wherein the three genes comprise ESR1 and two genes selected from DAPK, APC, TIMP-3, CALCA and RAR-beta.

7. The method according to any one of claims 1 to 4 wherein the method comprises determining the RNA expression level of a total of four genes.

8. The method according to claim 7 wherein the four genes comprise ESR1 and three genes selected from DAPK, APC, TIMP 3, CALCA and RAR-beta.

9. The method according to any one of claims 1 to 4 wherein the method comprises determining the RNA expression level of a total of five genes.

10. The method according to claim 9 wherein the five genes comprise ESR1 and four genes selected from DAPK, APC, TIMP-3, CALCA and RAR-beta.

11. The method according to any of the preceding claims wherein the sample is a cervical scraping.

12. A kit for use in detecting susceptibility to, or the incidence of, cervical cancer in a cervical sample from a patient comprising;
a) gene specific primers for determining the RNA expression level of the ESR1 gene; and
b) an Ayre's spatula and/or an endocervical brush for removing cervical cells from the patient.

13. The kit according to claim 12 further comprising a gene specific probe for the ESR1 gene.
